# EUROPEAN PATENT SPECIFICATION

(11) **EP 3 945 829 B1**
(45) Date of publication and mention of the grant of the patent: **20.08.2025**
(21) Application number: 20721978.3
(22) Date of filing: 30.03.2020
(51) Int. Cl.: A01N 63/34, A01N 25/00, A01N 25/08, A01N 25/22, A01P 3/00, C12N 1/14

(54) **AFLATOXIN BIOCONTROL COMPOSITION**
AFLATOXIN BIOKONTROLLZUSAMMENSETZUNG
COMPOSITION DE LUTTE BIOLOGIQUE CONTRE L'AFLATOXINE

(30) Priority: 02.04.2019 US 201962828453 P
(43) Date of publication of application: 09.02.2022
(73) Proprietor: CORN Products Development Inc., Westchester, IL 60154 (US); United States Department of Agriculture, Beltsville, Maryland 20705-5131 (US)
(72) Inventor: MEHTA, Pushpak, Bridgewater, New Jersey 08807 (US); MACRANDER, Mark, Bridgewater, 08807 (US); COTTY, Peter, Beltsville, Maryland 20705 (US)
(74) Representative: Novagraaf Group
(86) International application number: PCT/US2020/025767
(87) International publication number: WO 2020/205764

(56) References cited:
- WO-A1-2014/191917
- CN-A- 105 231 138
- US-A1- 2010 223 693
- US-B1- 7 361 499
- "Agricultural Sustainability", 1 January 2013, ELSEVIER, ISBN: 978-0-12-404560-6, article NADINA MÜLLER-FISCHER: "Nutrient-focused Processing of Rice", pages: 197 - 220, XP055702547, DOI: 10.1016/B978-0-12-404560-6.00010-1
- JAY E. MELLON ET AL: "Substrate Utilization by Aspergillus flavus in Inoculated Whole Corn Kernels and Isolated Tissues", JOURNAL OF AGRICULTURAL AND FOOD CHEMISTRY, vol. 53, no. 6, 1 March 2005 (2005-03-01), US, pages 2351 - 2357, XP055702561, ISSN: 0021-8561, DOI: 10.1021/jf040276g
- ZUZANA HRUSKA ET AL: "Temporal Effects on Internal Fluorescence Emissions Associated with Aflatoxin Contamination from Corn Kernel Cross-Sections Inoculated with Toxigenic and Atoxigenic Aspergillus flavus", FRONTIERS IN MICROBIOLOGY, vol. 8, 15 September 2017 (2017-09-15), XP055703460, DOI: 10.3389/fmicb.2017.01718

## Description

This Application claims priority to US Provisional Patent Application Number 62/828,453, filed April 2, 2019.

This invention was made in collaboration with the United States Department of Agriculture (USDA) under Agreement No. FAIN 58-6054-8-008. The United States Government has certain rights in this invention.

The present disclosure relates to compositions and methods comprising use of corn germ for utilizing atoxigenic *Aspergillus* genotypes to control aflatoxin-producing *Aspergillus* genotypes on crops and in the environment.

Aflatoxins, produced by the plant-pathogen *Aspergillus flavus,* are potent carcinogens that may contaminate many cereals, legumes, nuts and other cultivated and non-cultivated foods and feeds, as well as the environment. Many developed countries strictly enforce regulations restricting aflatoxin concentrations in food and feed. Many naturally occurring *Aspergillus flavus* genotypes do not have the capacity to produce aflatoxins. These genotypes that do not produce aflatoxins are typically referred to as "atoxigenic". Local atoxigenic genotypes can be used to displace and thus reduce the frequencies of aflatoxin producers on crops, in soil, and throughout the environment. In fact, the most effective method to limit aflatoxin contamination in crops is through the use of such beneficial atoxigenic genotypes. This type of aflatoxin management has been used commercially in many countries, including the USA, Italy, Nigeria, and Kenya.

For best management, atoxigenic genotypes must be actively growing in the field prior to the time when aflatoxin-producing fungi rapidly increase. To deliver and support the growth of active ingredient beneficial atoxigenic fungi in agricultural fields, these fungi are coated on a nutrient-supplying carrier such as cereal grains (wheat, sorghum, barley). After application, when environmental conditions favor reproduction of *A. flavus,* the beneficial fungi grow and reproduce on the nutrient carrier. The spores produced during this period disperse to and protect the crop by displacing and competing with aflatoxin producers. Quick sporulation and spreading over soil, within-field organic resources, and crop surfaces is essential for optimal ability of atoxigenic genotypes to out-compete and displace the harmful aflatoxin producing *A. flavus.* To be most effective, atoxigenic genotypes must increase frequencies to become dominant components of *A. flavus* communities. Dominance is best achieved by growth and spore production by the atoxigenic strains prior to the time when aflatoxin producing strains begin rapid increases on crop biomass and other organic resources in the field. Initial colonization of organic resources in agricultural fields drives a type of founder effect. Thus, faster sporulation facilitates efficacy by covering soil and crop surfaces with beneficial atoxigenic strains. However, grain-based biocontrol products may take a few days to establish and sporulate. Furthermore, grains may be eaten by birds or insects before spores are produced. Slower and less spore production may influence overall product effectiveness.

Manufacturing from whole grains forces the product to conform physically to the grain. For example, large grains result in product with large particle size. Large particle size means fewer particles per gram and few points of fungal release in treated fields. The current invention includes germ purification and particle sizing that allows consistent product size over time and deliberate management of particle size to enhance flowability and to optimize the number of product particles per mass and thus flexibility to manage the number of particles per weight delivered to treated fields in order to optimize coverage of target crops.

Additionally, producing biocontrol formulations with cereal grains (wheat, sorghum, barley) involves several steps. The grain must be purchased, transported to the facility, stored, pasteurized, and cooled before being coated with the beneficial atoxigenic spore suspension. These steps require more labor, more manufacturing space, extensive capital investment in equipment, and more energy, which leads to high operating costs. Disclosed herein is a beneficial new aflatoxin biocontrol composition that enables faster and greater sporulation of beneficial atoxigenic strains for aflatoxin biocontrol, while effectively reducing labor, cost, manufacturing space, and/or energy consumption.

The invention relates to an agricultural biocontrol composition according to claims 1-5. The agricultural biocontrol composition comprises a nutrient carrier and an atoxigenic strain of *Aspergillus,* wherein the nutrient carrier comprises corn germ, and wherein the atoxigenic strain of *Aspergillus* is coated on the surface of the corn germ. In still other embodiments, the nutrient carrier is corn germ. In some embodiments, the atoxigenic strain of *Aspergillus* is an *Aspergillus oryzae* strain, an *Aspergillus flavus* strain, an *Aspergillus sojae* strain, or a mixture thereof. In still yet even further embodiments, the atoxigenic strain of *Aspergillus* is an *Aspergillus flavus* strain. In yet other embodiments, the agricultural biocontrol composition further comprises one or more elements suitable for the biocontrol purpose, such as those selected from the group consisting of a carrier agent, a spreading agent (spreader), a binding agent, an osmoprotectant, a colorant, and a preservative.

In some embodiments, the agricultural biocontrol composition comprises a seed binder to help the atoxigenic strain of *Aspergillus* stick to the corn germ. In further embodiments, the seed binder comprises a polymer, such as a polymer compatible for the purpose of sporulating an atoxigenic strain of *Aspergillus.* The atoxigenic strain of *Aspergillus* is coated on the surface of the corn germ. In some embodiments, the atoxigenic strain of *Aspergillus* is coated on the surface of the corn germ using a polymer.

In some embodiments, the corn germ is produced as a by-product of a corn wet-milling process. In other embodiments, the corn germ is produced through a process other than wet-milling. In some embodiments, the corn germ is produced by a process that does not comprise pearling, roasting, and/or steaming. In some embodiments, the produced corn germ is size sorted with sieving and/or other means. In some embodiments, a set of sieves of specified sizes are used to remove smaller and larger pieces and provide a desired size of germ particles.

In some embodiments, the agricultural biocontrol composition is produced by a process that does not comprise the step of devitalizing, pearling or rolling, sterilizing by roasting, and/or cooling the corn germ before said corn germ is combined with or coated with the atoxigenic strain of *Aspergillus.* In some embodiments, the agricultural biocontrol composition is essentially free of fungi other than the atoxigenic strain of *Aspergillus,* and essentially free of disease-causing enterobacteria. In some embodiments, the agricultural biocontrol composition comprises equivalent or less bacteria compared to a composition consisting essentially of corn germ produced by a corn wet-milling process.

The invention relates to a method according to claims 6-11 for producing an agricultural biocontrol composition comprising a nutrient carrier and an atoxigenic strain as defined above and in claims 1-5. Some embodiments are directed to a method for producing an agricultural biocontrol composition that is essentially free of fungi other than the atoxigenic strain of *Aspergillus,* and essentially free of disease-causing enterobacteria. Still other embodiments are directed to a method for producing an agricultural biocontrol composition that introduces equivalent or less bacteria compared to a corn wet-milling process. The method comprises obtaining corn germ. In some embodiments, the method comprises obtaining the corn germ from a corn wet-milling process. In other embodiments, the method comprises obtaining the corn germ from a process other than a corn wet-milling process. In even further embodiments, the method does not comprise pearling, roasting, and/or steaming the corn germ. The method comprises combining the corn germ with an atoxigenic strain of *Aspergillus* to produce the agricultural biocontrol composition. In still other embodiments, the method comprises coating the corn germ with the atoxigenic strain of *Aspergillus.* In some embodiments, the method does not comprise a step of devitalizing, sterilizing by roasting, and/or cooling of the corn germ before said corn germ is either combined with or coated with the atoxigenic strain *of Aspergillus.* In yet other embodiments, the method comprises sieving the corn germ to remove broken pieces produced during the corn wet-milling process. In even further embodiments, the method comprises sieving with a US Sieve size No. 5 (5 Mesh) or size No. 6 (6 Mesh) to remove larger pieces and trash, and/or US Sieve size No. 7 (7 Mesh) or No. 8 (8 Mesh) to remove smaller pieces by allowing smaller pieces through said sieve. In some embodiments, the sieve is selected to provide any desired particle size range or particle size of the nutrient carrier that is beneficial for use in the desired agricultural biocontrol composition. In some embodiments, the method further comprises combining the sieved corn germ with an atoxigenic strain of *Aspergillus* to produce the agricultural biocontrol composition. In other embodiments, the method comprises coating the sieved corn germ with an atoxigenic strain of *Aspergillus* to produce the agricultural biocontrol composition.

In some embodiments, the agricultural biocontrol composition described herein supports quick sporulation of the atoxigenic strain *of Aspergillus* within about 48 hours after the agricultural biocontrol composition is applied under conditions suitable for sporulation of the atoxigenic strain of *Aspergillus.* In some embodiments, the agricultural biocontrol composition described herein provides at least 2 times more spores than compositions and methods that use grain as a nutrient carrier under the same conditions, within 36 hours, 48 hours, 60 hours, 72 hours, 84 hours, 96 hours, 108 hours, 120 hours, 132 hours, 144 hours, 156 hours, or 168 hours after sporulation begins.

The invention also relates to a method for controlling aflatoxin contamination in an agricultural plant or an agricultural product derived from the plant, according to claims 12-13.

The method comprises applying an aflatoxin-reducing effective amount of the agricultural biocontrol composition described herein to a plant, locus of growth or plant product.

The invention further relates to a method for controlling aflatoxin contamination in a cultivated area according to claims 12-13. The method comprises applying an aflatoxin-reducing effective amount of an agricultural biocontrol composition described herein to a cultivated area.

### BRIEF DESCRIPTION OF THE FIGURES

**Figures 1A and 1B** depict growth and spore production by an atoxigenic *Aspergillus flavus* genotype used in a biological control product on germ (A) and Sorghum (B) after 72 hours at 31°C and 100% humidity.
**Figure 2** depicts spore yield comparison as measured by Nephelometric Turbidity Unit (NTU) using sorghum and corn germ. Measurements were taken from 0-168 hours after inoculation.
**Figure 3** depicts average spore yield as measured by NTU using sorghum and corn germ, in three replicates. Measurements were taken from 0-168 hours after placing the biocontrol products under conditions favorable for growth and release of the active ingredient.

Corn germ is disclosed herein as a superior nutrient carrier for use in an agricultural biocontrol composition that also contains an atoxigenic strain and is used to control aflatoxin, producing more spores than a variety of commercially available biocontrol compositions that use other nutrient carriers, e.g., cereal grains. Compositions and methods for quick sporulation, which provides more effective aflatoxin reduction, are also described herein. Corn germ produced as a co-product of starch manufacturing and further improved for flowability and product quality (removing fines, pericarp and foreign material and fragments) surprisingly allows for rapid spore production and release and is a very good nutrient carrier. In addition, the germ has a microbiology profile that both regulators and food industry consider safe for crops, environment and consumers. As such, corn germ produced during the starch manufacturing process can be improved for use in production of biocontrol compositions after sorting to remove undesirable components, improve particle uniformity, and increase flowability. Thus, a process is described herein for producing an improved germ product that facilitates the production of an agricultural biocontrol composition and eliminates devitalizing, sterilizing by roasting, cooling steps etc. commonly required to produce other commercially available agricultural biocontrol compositions. Since these steps (e.g., devitalizing, roasting, cooling etc) are normally involved in biocontrol products made with grain (e.g., wheat, barley, or sorghum), the methods described herein significantly reduce initial equipment capital as well as operating costs. Compositions and methods using the improved corn germ described herein also eliminate steps of procuring, transporting, and storing grain, as corn germ is a co-product of a starch wet-milling plant.

Accordingly, the invention relates to agricultural biocontrol composition comprising an atoxigenic strain of Aspergillus, and a nutrient carrier, wherein the nutrient carrier comprises corn germ, and wherein the atoxigenic strain of Aspergillus is coated on the surface of the corn germ. As used herein the following terms are synonymous with "atoxigenic": non-toxigenic, non-aflatoxigenic and non-aflatoxin.

An atoxigenic strain *of Aspergillus* can be used to displace and thus reduce the frequencies of aflatoxin producers on crops, in soil, and throughout the environment. Any atoxigenic strain of *Aspergillus* known in the art can be used, including but not limited to an *Aspergillus oryzae* strain, an *Aspergillus flavus* strain, an *Aspergillus sojae* strain, an *Aspergillus parasiticus* strain, or a mixture thereof. In other embodiments, the atoxigenic strain of *Aspergillus* is *Aspergillus flavus.* In still other embodiments, the atoxigenc strain of *Aspergillus* is *Aspergillus oryzae.* In even further embodiments, the atoxigenc strain of *Aspergillus* is *Aspergillus sojae.* In yet even further embodiments, the atoxigenc strain of *Aspergillus* is *Aspergillus parasiticus.*

In some embodiments, atoxigenic *Aspergillus flavus* strains include, but are not limited to *A. flavus* strains AF36 (NRRL 18543), CT3, K49 (NRRL 30797), La3279, La3304, Ka16127, Og0222, PKM03-N, ARS Culture Collection deposit numbers NRRL 50427, NRRL 50428, NRRL 50429, NRRL 50430, NRRL 50431, NRRL 18543 (AF36 in Prevail^{™}, Arizona Cotton Research and Protection Council), NRRL 21882 (AflaGuard^{™}, Syngenta), and Fungal Genetics Stock Center (FGSC) deposit numbers FGSC A2223, FGSC A2220, FGSC A2226, and FGSC A2229 (*i.e*., four atoxigenic genotypes in FourSure^{™}, Texas Corn Producer's Board).

Additional *Aspergillus* species and strains that can be used are described in U.S. Patent Nos. 9011891, 8637002, 5171686, 5294442, 7361499, 6306386, and 6027724, and Ehrlich et al. (2004, Applied Microbiology & Biotechnology 65: 473-478), Probst et al. (2010, Plant Disease 95 (2), 212-218), Brown et al. (1991, J. Food Protection 54, 623-626), Cotty (1994a, Phytopathology 84, 1270-1277), Cotty, P. J. (1994b, Mycopathologia 125, 157-162), Probst et al., (2011, Pant disease 95:212-218), Bandyopadhyay et al. (2016, World Mycotoxin J 9, 771-789), and Mehl (2012, Ann. N. Y. Acad. Sci. 1273, 7-17).

Any atoxigenic *Aspergillus* species and strains can be used in one or more agricultural biocontrol compositions of the invention. The methods and compositions of the invention have been successfully tested using different, distinct genotypes of atoxigenic *Aspergillus flavus,* and in each case faster sporulation can be observed using an agricultural biocontrol composition described herein, compared to grain based methods and compositions.

In some embodiments, the atoxigenic strain *of Aspergillus* is selected based on one or more factors, including but not limited to geographical location of the field, environmental conditions, microbiological conditions of the field, chemical conditions of the field, weather conditions of the location, etc. For example, an atoxigenic strain of *Aspergillus* can be particularly selected as it has evolved to thrive in the growing conditions associated with the geography at issue.

In addition, there are products fully registered in several African countries including Nigeria, Senegal, Burkina Faso, Ghana, and Kenya, which utilize four additional genotypes of *A. flavus* as active ingredients. These products are called either Aflasafe or Aflasafe with a country specific code (e.g. Aflasafe KE01 for Kenya). For example, Aflasafe NG01 applies a coating containing all four genotypes to sorghum grain. The four genotypes in NG01 are endemic in maize producing regions of Nigeria and are well adapted to those regions. In addition, there are products developed for several other countries that are in the second or third year of farmer field trials and all have shown good efficacy in altering the fungal communities resident in agricultural fields. Each of these additional products are manufactured in the same manner as Aflasafe and *Aspergillus flavus* AF36 Prevail. These products each consist of atoxigenic strains of *A. flavus* native to the target regions and are manufacturing by coating a conidial suspension consisting of equal proportions of all four isolates onto roasted sorghum grain, as explained in the manufacturing section below.

Fungi used as active ingredients in the biocontrol products can be characterized by one or more physiological, phenotypic, molecular and/or genotypic testing methods well known to those skilled in the art. Examples of suitable tests for strain characterization include, but are not limited to, random amplified polymorphic DNA (RAPD)-based characterization, polymerase chain reaction (PCR) assays/product patterns, DNA fingerprinting, AFLP markers, and simple sequence repeats (SSR).

In some embodiments, the best strain(s) of atoxigenic *Aspergillus* to use in any specific agronomic or horticultural situation are chosen for a specific growing region and/or plant species. Thus, one can select a biocontrol strain that has evolved to thrive in the growing conditions associated with the geography where it is used according to the methods and compositions described herein. One embodiment is directed to an agricultural biocontrol composition comprising, consisting, or consisting essentially of corn germ, optionally purified, that is associated with an atoxigenic *Aspergillus* adapted to a particular growing region.

The methods and compositions described herein are contemplated to work for all filamentous fungi capable of utilizing nutrients saprophytically and applied to agricultural fields and/or the environment with the intention of reproduction and dispersal. This includes biocontrol fungi such as *Trichoderma spp., Beauveria spp., Metarhizium spp., Aspergillus spp., Fusarium spp., Cladosporium spp.,* and many others known and not yet described. Such compositions and methods are not according to the invention.

Corn germ is normally pleasantly nutty in taste and rich in oil. Previously, it is mainly used for extraction of maize oil and manufacturing of feed supplements (e.g., corn germ meal). In some embodiments, corn germ is produced in a starch purification process. In some embodiments, the starch purification process is a wet milling process or a dry milling process. In some embodiments, corn germ can be produced by other methods, such as those described in U.S. Patent Nos. 2459548, 2282817, 4341713, 4495207, 5297348, and 6899910.

*Corn germ and wet-milling process as used within the invention*
*(corn germ and the wet milling process alone are not part of the invention).*

A mature corn kernel is composed of four major parts, namely endosperm, germ, pericarp, and tip cap. Corn germ, or maize germ, is the reproductive part of corn that germinates to grow into a plant. As used herein, corn germ is synonymous with the embryo and closely associated tissues of the corn seed or corn kernel. The corn germ used in the methods and compositions described herein are incapable of germinating or growing into a corn plant due to the process by which the corn germ is obtained, which is a component of the current invention. In some embodiments the corn kernels may lack the ability to germinate prior to processing as a result of one or more physical, physiological, phenotypical, molecular and/or genotypic abnormalities or deficiencies that prevent, inhibit or otherwise impede its ability to germinate or grow. In some embodiments, the corn germ for making the agricultural biocontrol compositions described herein is obtained from corn grains. In some embodiments, the corn germ is produced as a co-product of a corn wet-milling process. The corn wet-milling is a process of breaking corn kernels into their component parts. It uses water and a series of steps to separate the parts to be used for various products. The process is based on physical separation of components, mostly by weight and size, while some chemicals such as aqueous sulphur dioxide (SO₂) may be used in certain steps (e.g., during the steeping process). In some embodiments, harvested corn grains are inspected before they are milled to eliminate contaminated corn grains.

In some embodiments, harvested corn grains are cleaned before they are milled. The cleaning step is used to sieve, separate particles by shape, density, and magnetic force to remove impurities. In some embodiments, a dockage tester with appropriate sieve number can be used to remove particles other than the corn grains, such as cob pieces, broken corn, foreign seeds, metal pieces, leaves, dirt, etc. In some embodiments, the corn is cleaned by a dry process, a wet process, or by both. In some embodiments, the cleaned corn grains are analyzed using an NIR spectrometer.

In one embodiment, the corn grain is hydrated prior to milling to loosen starch granules from the protein matrix and to make germ resilient to milling. Germ density is reduced and the kernel is softened which makes the milling easier. In some embodiments, sulphur dioxide (concentration ranging between 300 to 2000 ppm) and/or lactic acid are added to the water. Sulphur dioxide can weaken the matrix allowing starch granules to separate out cleanly, while lactic acid breaks down the endosperm protein matrix and helps in better separation of starch. Lactic acid or sulphur dioxide also lowers pH which, in combination with high temperature, prevents growth of undesirable microbes. In some embodiments, cleaned corn grain is steeped in a large tank with water. In some embodiments, the steeping process is carried out at a temperature of about 120-130 °F (48.9-54.4 °C). In some embodiments, the steeping process lasts about 40 hours. Afterwards, the steepwater can be drained.

In some embodiments, germ is separated from the other parts of the corn. The corn that has been steeped is then sluiced to a grind mill which breaks open the kernel of corn and releases the germ particle. In some embodiments, an attrition mill such as a disk mill can be used. In other embodiments, the grinding is slow and the elements used to grind are blunt to ensure intact removal of germ. This slurry is then sent through a series of germ cyclones to separate the germ from the remaining fiber, gluten, and starch. Germ particles are lighter than the remaining corn components and a higher fraction of the germ will float to the overflow of the germ cyclones. The germ purity (% oil) is then controlled by properly adjusting the density of the system and the amount of overflow on each stage of the germ cyclones. In some embodiments, the germ separation step comprises recovering germ as intact as possible. In some embodiments, corn germ is separated from fiber, starch, protein and water using a series of screens, centrifuges, and/or cyclones. In some embodiments, during a wet-milling operation, the fraction containing germ (e.g., whole germ, essentially intact germ, and/or partially grinded germ) is selected and separated. The germ is then sent through a germ press to lower the moisture to 50%. In some embodiments, this germ is then sent through germ dryers where the moisture is lowered to less than 5%. These dryers typically run above 93.3 °C (200 °F) and the retention time of germ in the dryers is 20-50 mins. The high temperature of the germ in the dryers helps inhibit microbial growth. The germ is then cooled to about 37.8 °C (100 °F) and stored.

It should be noted that the above-mentioned process is not a limiting one, and other modified wet-milling processes or different milling processes for corn can be used.

In some embodiments, the corn germ that is used to make the biocontrol composition described herein is produced as a flowable portion of a milling process. In some embodiments, such flowable portion of the milling process is separated from the unflowable portions (e.g., contaminants) by a sieving process. Some portions produced during the process of making corn germ supports sporulation and others do not. In some embodiments, the sieving process is used to separate the portions that are capable of supporting sporulation (i.e., flowable portions) from the portions that are not capable of supporting sporulation (i.e., unflowable portions). In some embodiments, the sieving process is also used to remove undesirable fragments and trash, and to provide a germ particle of desired size for the specific application. Flowability is the ability of granular solids and powders to flow during discharge from transportation or storage containments. Flowability is affected by the natural properties of a material, but also by several interacting environmental factors, such as material moisture, storage temperature, particle size distribution, relative humidity, time, compaction of the material mass, vibrations during transport, variations throughout storage process, chemical composition of the material, as well as the addition of flow agents. In some embodiments, the germ is further processed before it is used to make the biocontrol composition. In some embodiments, the corn germ produced is sieved to remove broken pieces produced during the corn wet-milling process, before the germ is coated with an atoxigenic strain of *Aspergillus.* In some embodiments, a set of sieves is used to remove smaller and larger pieces and provide a desired size of germ particles. In some embodiments, the step of sieving comprises using a US Sieve size No. 5 (5 Mesh) or size No. 6 to remove larger pieces and trash, and/or US Sieve size No. 7 (7 Mesh) or No. 8 to remove smaller pieces by allowing smaller pieces through.

However, in some embodiments, smaller germ particle size is advantageous for compatibility with application methods and/or to allow for more particles per pound and thus more points for distribution of the biocontrol atoxigenic strain after application. Particle size may also be adjusted larger or smaller in order to alter the percent of product held up in the crop canopy after application depending on the intent, the target crop, and environmental conditions. In some embodiments, purified components of the germ are used as sized nutritional particles that are compatible with delivery of filamentous fungi and other microbials. Some embodiments allow for adjustment of particle size to desired parameters in order to best meet the objectives of the target application.

In addition to sieving, other processes can also be used, such as any inexpensive process for separating unflowable portions (contaminants) from flowable portions, such as a continuous process, as long as the right particulates are selected, which are nutritive to the atoxigenic *Aspergillus* strain.

In some embodiments, corn germ obtained through a process as described herein has a microbiology profile that is acceptable to a regulatory agency, such as (Environmental Protection Agency) EPA and United State Department of Agriculture (USDA). In some embodiments, the germ is free of unwanted fungi and bacteria. For example, prior to biocontrol the germ is free of toxigenic *Aspergillus* species, free of fungi other than a beneficial atoxigenic *Aspergillus* species, and free of disease-causing enterobacteria. In some embodiments, the germ is essentially free of unwanted fungi and bacteria. As used herein, the term "essentially free" means the level of the unwanted fungi and bacteria is not detectable, or below the standard set by a regulatory agency, such as EPA, Food and Drug Agency (FDA) or USDA, or otherwise required by law and/or regulations. For example, in some embodiments, the corn germ is processed at conditions sufficient to ensure food safety as required by the U.S. Food Safety Modernization Act.

Since corn germ obtained through a process as described herein is not viable, and it has an acceptable microbiology profile, unlike sorghum or other grain as nutrient sources for supporting the growth of an atoxigenic strain, corn germ does not have to be sterilized to kill pathogens, or to be devitalized (e.g., by roasting). Accordingly, methods disclosed herein for preparing corn germ and for making a biocontrol composition do not comprise a step of devitalizing (e.g., by roasting), sterilizing, and/or cooling of corn grains or the corn germ before the corn germ is combined with or coated with the atoxigenic strain of *Aspergillus.*

### Formulation

When a grain (e.g., sorghum, wheat, or barley) is used as a nutrient carrier, one must devitalize by removing seed coat (as in barley) or by roasting (as in sorghum) and coat the grain with spores of the beneficial atoxigenic *Aspergillus* strain, which contributes to production costs, product fragility, and generation of spore dust (resulting in loss of spore material that could be applied to a field). It also inevitably leads to spore contamination, due to loss of control over where spores go. The methods described herein for producing the agricultural biocontrol composition described herein do not suffer from these problems.

Without wishing to be bound by any particular theory, faster sporulation is useful for the efficacy of an agricultural biocontrol composition. In fact, just having a nutrient source does not necessarily mean that the *Aspergillus* strain will produce spores rapidly. The compositions and methods described herein allow the atoxigenic strain to produce spores rapidly, and further allow the spores to be produced on an adequately uniform particle size (e.g., produced through a fractionation/sieving process). The methods described herein enable the material to be flowable, with more spore yield and/or faster spore production. Also, the methods described herein allow the biocontrol composition to be produced at a lower cost.

Spores should be released as quickly as possible to ensure effective taking-over by the atoxigenic strain in the system. Nutrient carriers using crop grains (e.g., wheat, sorghum, or barley) are often eaten and therefore have less in-field time compared to corn germ. In addition, corn germ is surprisingly much better than grains (e.g., sorghum) at quickly releasing the spores. This is particularly beneficial, as farmers may not apply the biocontrol product early enough in time to their fields to enable the sorghum product to sporulate, so biocontrol products using corn germ increases the likelihood of successfully controlling toxigenic *Aspergillus* species in the fields.

Agricultural biocontrol compositions of the present invention can be formulated as needed. In some embodiments, the biocontrol compositions comprise one, two, three, four, five, six, seven, eight, nine, ten, or more atoxigenic strains of *Aspergillus,* such as strains of *Aspergillus flavus.*

The biocontrol compositions further comprise a nutrient carrier. The nutrient carrier comprises corn germ. In some embodiments, the nutrient carrier comprises substantially pure corn germ. In some embodiments, the nutrient carrier consists essentially of corn germ. In some embodiments, the nutrient carrier consists of corn germ.

The nutrient carrier comprises corn germ. In some embodiments, the nutrient carrier comprises an effective amount of corn germ for rapid sporulation of an atoxigenic *Aspergillus* strain, such as an atoxigenic *Aspergillus flavus* strain. As used herein, the term "effective amount" refers to an amount of corn germ that allows production of about 1x10⁶, 2x10⁶, 3x10⁶, 4x10⁶, 5x10⁶, 6x10⁶, 7x10⁶, 8x10⁶, 9x10⁶, 1x10⁷, 2x10⁷, 2x10⁷, 3x10⁷, 4x10⁷, 5x10⁷, 6x10⁷, 7x10⁷, 8x10⁷, 9x10⁷, 1x10⁸, 2x10⁸, 3x10⁸, 4x10⁸, 5x10⁸, 6x10⁸, 7x10⁸, 8x10⁸, 9x10⁸, 1x10⁹, 1x10⁹, 2x10⁹, 3x10⁹, 4x10⁹, 5x10⁹, 6x10⁹, 7x10⁹, 8x10⁹, 9x10⁹, or more spores per gram of the nutrient carrier, within about 24 hours, 36 hours, 48 hours, 60 hours, 72 hours, 84 hours, 96 hours, 108 hours, 120 hours, 132 hours, 144 hours, 156 hours, or 168 hours after sporulation begins.

In some embodiments, the nutrient carrier comprises corn germ at an amount of at least about 5%, 10%, 15%, 20%, 25%, 30%, 35%, 40%, 45%, 50%, 55%, 60%, 65%, 70%, 75%, 80%, 85%, 90%, 95%, or 100% of the nutrient carrier, as measured by weight.

In some embodiments, a nutrient carrier of the present disclosure consists essentially of corn germ. As used herein, the term "consists essentially of" means the nutrient carrier may contain other ingredient(s) that do not materially affect the characteristics of the biocontrol compositions disclosed herein. For example, the other ingredient(s) is presented at a level that does not materially affect the ability of the nutrient carrier to induce rapid sporulation of an atoxigenic *Aspergillus* strain, so that at least about 1x10⁶, 2x10⁶, 3x10⁶, 4x10⁶, 5x10⁶, 6x10⁶, 7x10⁶, 8x10⁶, 9x10⁶, 1x10⁷, 2x10⁷, 2x10⁷, 3x10⁷, 4x10⁷, 5x10⁷, 6x10⁷, 7x10⁷, 8x10⁷, 9x10⁷, 1x10⁸, 2x10⁸, 3x10⁸, 4x10⁸, 5x10⁸, 6x10⁸, 7x10⁸, 8x10⁸, 9x10⁸, 1x10⁹, 1x10⁹, 2x10⁹, 3x10⁹, 4x10⁹, 5x10⁹, 6x10⁹, 7x10⁹, 8x10⁹, 9x10⁹, or more spores are produced per gram of the nutrient carrier, within about 24 hours, 36 hours, 48 hours, 60 hours, 72 hours, 84 hours, 96 hours, 108 hours, 120 hours, 132 hours, 144 hours, 156 hours, 168 hours after sporulation begins.

In some embodiments, the nutrient carrier consists of corn germ.

Corn germ can be produced by any method known to a person of skill in the art. In some embodiments, corn germ produced in a milling process can be used as the nutrient carrier to support sporulation and/or propagation of a beneficial atoxigenic *Aspergillus* strain. In some embodiments, corn germ produced in a wet milling process is used. In some embodiments, corn germ produced in a process other than wet milling is used.

In some embodiments, the corn germ used as a nutrient carrier in an agricultural biocontrol composition has a moisture content of about 3%, 4%, 5%, 6%, 7%, 8%, 9%, 10%, 11%, 12%, 13%, 14%, 15%, or more, such as about 4% to 5%, or about 8% to 10%.

In some embodiments, the agricultural biocontrol composition comprises about 1x10⁶, 2x10⁶, 3x10⁶, 4x10⁶, 5x10⁶, 6x10⁶, 7x10⁶, 8x10⁶, 9x10⁶, 1x10⁷, 2x10⁷, 3x10⁷, 4x10⁷, 5x10⁷, 6x10⁷, 7x10⁷, 8x10⁷, 9x10⁷, 1x10⁸, 2x10⁸, 3x10⁸, 4x10⁸, 5x10⁸, 6x10⁸, 7x10⁸, 8x10⁸, 9x10⁸, 1x10⁹, 2x10⁹, 3x10⁹, 4x10⁹, 5x10⁹, 6x10⁹, 7x10⁹, 8x10⁹, 9x10⁹, 1x10¹⁰, 2x10¹⁰, 3x10¹⁰, 4x10¹⁰, 5x10¹⁰, 6x10¹⁰, 7x10¹⁰, 8x10¹⁰, 9x10¹⁰, 1x10¹¹, 2x10¹¹, 3x10¹¹, 4x10¹¹, 5x10¹¹, 6x10¹¹, 7x10¹¹, 8x10¹¹, 9x10¹¹, 1x10¹², 2x10¹², 3x10¹², 4x10¹², 5x10¹², 6x10¹², 7x10¹², 8x10¹² , 9x10¹² or more spores of one or more atoxigenic *Aspergillus* strains per kg before or after the composition is applied.

In some embodiments, the agricultural biocontrol compositions further comprise a binding agent, such as an agriculturally acceptable binding agent. In some embodiments, spores of an atoxigenic *Aspergillus* strain, particularly spores in high quality, are attached to the corn germ with the help of a compatible seed binder (a.k.a., seed coater, seed sticker, or coating binder). In some embodiments, the seed binder comprises a polymer. An example of a suitable polymer is Milliken^{®} Treating Solutions Green Polymer 3118, but many polymers and seed colorants used in the seed industry would serve equally well. In some embodiments, the polymer is presented in a sufficient amount to help the spores spread across the germ surface, stick to the germ, and facilitate germ flow after the germ has been coated.

In some embodiments, the seed binder is composed of one or more polymers. In some embodiments, the polymer is a synthetic polymer, co-polymers, natural biopolymers, or a combination thereof. In some embodiments, a colorant is added to the polymer.

In some embodiments, the polymer used in the agricultural biocontrol compositions is a non-toxic polymer. As used herein, the term "non-toxic" means the polymer does not have any negative impact on, or does not have significant impact on the viability of atoxigenic *Aspergillus* strains (e.g., the sporulation speed of the atoxigenic *Aspergillus* strain is not slowed down by the polymer for more than 5% compared to the conditions without the polymer).

In some embodiments, the seed binder comprises a polymer made by reaction of the same monomers. In some embodiments, the seed binder comprises a co-polymer made by reaction of two or more different monomers. In some embodiments, the monomer for the polymer or the co-polymer is selected from the group consisting of ethylene glycol, vinyl acetates, vinyl alcohols, vinyl acrylates, hydroxyethyl acrylate, vinylpyrrolidones, acrylic acid, acrylamides and methylacrylamides.

In some embodiments, the seed binder comprises one or more polysaccharides. Polysaccharides that can be used in a biocontrol composition of the present disclosure include, but are not limited to, starch, dextrin, glycogen, cellulose, hemicellulose, polydextrose, inulin, beta-glucan, pectin, psyllium husk mucilage, galactomannans or gums (e.g., beta-mannan, carob, fenugreek, guar gum, tara gum, xanthan gum, konajc gum, gum acacia, karaya gum, tragacanth gum, arabinoxylan gum, gellan gum, and their derivatives), glucomannan, agar, agaropectin, agarose, alginate, carrageenan, chitin, chitosan, and mixtures thereof.

In some embodiments, the seed binder comprises one or more celluloses, such as carboxymethylcelluloses, carboxyethylcelluloses, hydroxymethylcelluloses, hydroxypropylcelluloses, methyl celluloses, hydroxy methyl propyl celluloses, dextrins, maltodextrins, and mixtures thereof.

In some embodiments, the seed binder comprises starch, modified starch, and mixtures thereof, such as those derived from corn, waxy maize, wheat, tapioca, waxy tapioca, potato, sorghum, rice, waxy rice and plant-based starch, flour and proteins (pulses). In some embodiments, modified starches include, but are not limited to, cationic starch, octenyl succinic anhydride starch, acetylated starch, propylene oxide treated starch, cross-linked starches such as with adipic acid, phosphorous oxychloride, and epichlorohydrin.

In some embodiments, the seed binder comprises fats, oils, proteins, polysaccharides, other derivatives of plants and animal products, and mixtures thereof.

More examples of seed binders can be found in U.S. Patent Nos. 7213367, 7189677, 5363754, 8273684, 7989391, 4349578, 5876739, 5763509, 3728817, 3671633, 4881343, 4853429, 6605268, 5737872, 6156699, 5849320, 5994265, 5344871, 5374670, 5506285, 6209259, 5163896, 8881453, 3905152, 4994013, 8931209, 10160692, 5967521, 4067141, 7223436.

Additional compounds can be used in the nutrient carrier in addition to corn germ, to increase sporulation, reduce rub off, improve flowability, alter appearance, or provide other benefits. Of particular interest are compounds capable of increasing sporulation of the atoxigenic *Aspergillus* strain within the initial stage, such as about 2 hours, 4 hours, 6 hours, 8 hours, 10 hours, 12 hours, 14 hours, 16 ours, 18 hours, 20 hours, 22 hours, 24 hours, 28 hours, 32 hours, 36 hours, 40 hours, 44 hours, 48 hours, 52 hours, 56 hours, 60 hours, 70 hours, or more after the agricultural biocontrol composition is placed under conditions suitable for sporulation. In some embodiments, the additional compound comprises corn oil or other vegetable oils, such as soybean oil or peanut oil.

In some embodiments, the agricultural biocontrol compositions further comprise an osmoprotectant, such as an agriculturally acceptable osmoprotectant. In some embodiment, the osmoprotectant is selected from, but not limited to, betaines, polyols, sugars and polyamines, such as glycine betaine, proline, ectoine, hydroxy-ectoine, glutamate, choline-o-sulfate, mannitol, maltose, trehalose, glycerol, sorbitol, and mixtures thereof.

In some embodiments, the agricultural biocontrol compositions further comprise an adhesive agent, such as an agriculturally acceptable adhesive agent. In some embodiments, a polymer in the seed binder described herein serves the purpose of the adhesive agent and reduces rub-off.

In some embodiments, the agricultural biocontrol compositions further comprise a stabilizer and/or a preservative, such as an agriculturally acceptable stabilizer and/or preservative.

In some embodiments, the agricultural biocontrol compositions further comprise a colorant, such as an agriculturally acceptable colorant.

In some embodiments, the formulation of the agricultural biocontrol compositions is a water dispersible formulation. In some embodiments, the formulation of the agricultural biocontrol compositions is a sprayable formulation.

Non-limiting examples of formulations for an agricultural biocontrol composition comprising an atoxigenic *Aspergillus* strain are described in U.S. Patent Nos. 5171686, 5294442, 6306386, 9011891, 9526240, 8173179, and 8734862.

In some embodiments, the agricultural biocontrol compositions further comprise a colorant. Any colorant suitable for agricultural purpose can be used. In some embodiments, the agricultural colorant is selected from those produced and marketed by Sun Chemical, BASF, Clariant, Keystone Aniline (Milliken), Chromatech Incorporated, Sensient Technologies, Aakash Chemicals, Organic Dyes and Pigments, AgriCoatings, ArrMaz, Retort Chemicals, and ER CHEM COLOR. In some embodiments, the seed binder contains color already, so no additional colorant is needed.
Since certain formulations used to apply an atoxigenic strain of *Aspergillus* species to soil may be considered a biopesticide by the EPA, in such instances, a formulation would be registered with EPA before commercial use. Accordingly, in some embodiments, a formulation of the present invention contains chemicals at a level acceptable to the EPA.

### Methods of using the agricultural biocontrol compositions of the invention

Using sorghum, wheat, barley or other grain as a nutrient source for the atoxigenic genotypes has unavoidable disadvantages, because one has to sterilize to kill pathogens associated with the grain, and then devitalize it by roasting and then cool in silo. Also, when sorghum or a grain is used in an agricultural biocontrol formulation and the formulation is distributed in field (e.g., by spraying), the grain will grow in the field and compete with the agricultural plants of interest. Since farmers do not want to worry about sorghum or other grains taking over the agricultural plants of interest, one has to sterilize (e.g., by roasting) and cooling sorghum grains before coating the grains with a beneficial *Aspergillus* strains. By replacing grain with corn germ, it is possible to avoid all of the above sterlizing, cooling, etc processing steps required for grain because corn germ is not viable and is essentially free of harmful pathogens, particularly when corn germ is produced by a widely used process, such as, for example, wet-milling. It is much less expensive to use corn germ because very few processing steps are required compared to that of sorghum or barley and the material is already generated by corn starch facilities for which production and use of agricultural biocontrol compositions may be advantageous. Accordingly, using corn germ reduces capital cost for manufacturing and thus reduces the costs for producing the agricultural biocontrol composition. This is very important to encourage farmers to apply the agricultural biocontrol composition to their fields and keep doing so every year reducing aflatoxins in crops and the environment throughout the production regions.

The agricultural biocontrol compositions can be used in any area contaminated by toxigenic *Aspergillus,* or for any plants (e.g., crops or other agriculturally important plants) susceptible to *Aspergillus* infection. Such plants include, but are not limited to, cereal grains (e.g., wheat, oats, rice, corn, barley, sorghum, rye, millet, triticale, amaranth, buckwheat, and quinoa), legumes (e.g., chickpeas, beans, peas, lentils, lupins, and mesquite), tree nuts (e.g., acorn, beech, breadnut, candlenut, chestnuts, deeknut, hazelnuts, almond, lola nut, kurrajong, mongongo, palm nuts, karuka, red bopple nut, apricot, cashew nut, betel, borneo tallow nuts, canarium nut, cashews, coconut, gabon nut, hickory, jack nuts, bread nuts, pekea nut, pistachio, and walnut), figs, peanuts, chili, and cotton.

In some embodiments, components of the agricultural biocontrol compositions such as corn germ, polymer, etc. are processed to be acceptable for application in organic farming. Organic farming is a holistic system designed to optimize the productivity and fitness of diverse communities within the agro-ecosystem, including soil organisms, plants, livestock and people. The principal goal of organic farming is to develop enterprises that are sustainable and harmonious with the environment. The general principles of organic production include, protect the environment, minimize soil degradation and erosion, decrease pollution, optimize biological productivity and promote a sound state of health, maintain long-term soil fertility by optimizing conditions for biological activity within the soil, maintain biological diversity within the system, recycle materials and resources to the greatest extent possible within the enterprise, provide attentive care that promotes the health and meets the behavioral needs of livestock, prepare organic products, emphasizing careful processing, and handling methods in order to maintain the organic integrity and vital qualities of the products at all stages of production, and rely on renewable resources in locally organized agricultural systems.

In some embodiments, the agricultural biocontrol compositions are mixed with seeds of an agricultural plant, and the mixture is stored in a container (e.g., a seed silo) before the mixture is utilized or fed to human or animals. This will control infection of toxic *Aspergillus* species in stored seeds, and elongate storage time.

In some embodiments, the agricultural biocontrol compositions are applied to an area susceptible or potentially susceptible to toxic *Aspergillus* species infection before, during, or after seeds of an agricultural plant are planted in the area. In some embodiments, the agricultural biocontrol compositions are sprayed on the field to prevent toxic *Aspergillus* species from taking root on the plants. In some embodiments, the agricultural biocontrol compositions are applied to the soil where plants are already growing to control toxic *Aspergillus* species in the soil or air or other plants and to reduce aflatoxin content in the target plants.

In some embodiments, the agricultural biocontrol compositions are applied to an area for cultivating a plant of interest before the plant is cultivated (e.g., when a seed of the plant is placed in the area; when an explant is placed in the area; when a grafting is made; when a plant is translocated to the area, when a growing season is coming, when a dormant plant regenerates, etc.). In some embodiments, the agricultural biocontrol compositions can be applied to the area about 4 hours, 8 hours, 12 hours, 16 hours, 20 hours, 24 hours, 28 hours, 32 hours, 36 hours, 40 hours, 44 hours, 48 hours, 60 hours, 70 hours, 80 hours, 90 hours, 100 hours, 1 week, 2 weeks, 3 weeks, 4 weeks, 2 months, 3 months, 4 months, 5 months, 6 months, 7 months, 8 months, 9 months, 10 months, 11 months, 12 months, 1.5 year, 2 years, 3 years, or more before the plant is cultivated. In some embodiments, the biocontrol composition can be applied more than one time before the plant is cultivated, such as about two times, three times, four times, five times, six times, or more before the plant is cultivated.

In some embodiments, the agricultural biocontrol compositions are applied to an area for cultivating a plant of interest when the plant is cultivated.

In some embodiments, the agricultural biocontrol compositions are applied to an area for cultivating a plant of interest after the plant is cultivated. In some embodiments, the agricultural biocontrol compositions can be applied to the area about 1 hour, 2 hours, 4 hours, 8 hours, 12 ours, 16 hours, 20 hours, 24 hours, 28 hours, 32 hours, 36 hours, 40 hours, 44 hours, 48 hours, 60 hours, 70 hours, 80 hours, 90 hours, 100 hours, 1 week, 2 weeks, 3 weeks, 4 weeks, 2 months, 3 months, 4 months, 5 months, 6 months, 7 months, 8 months, 9 months, 10 months, 11 months, 12 months, 1.5 year, 2 years, 3 years, 4 years, 5 years, 6 years, 7 years, or more after the plant is cultivated. In some embodiments, the agricultural biocontrol compositions can be applied more than one time after the plant is cultivated, such as about two times, three times, four times, five times, six times, seven times, eight times, nine times, ten times, or more after the plant is cultivated. In some embodiments, the agricultural biocontrol compositions are applied to the field depending on crop phenology. For example, in some embodiments, for perennial trees or crops, the agricultural biocontrol compositions can be applied biannually, annually, twice a year, three times a year, four times a year, five times a year, six times a year, or more, or as needed. In some embodiments, the agricultural biocontrol compositions can be applied during a time conducive to fungal growth, such as months with a warm temperature and/or a high moisture level (e.g., from May to August in Northern hemisphere). Without wishing to be bound by any particular theory, after application to the field and uptake of moisture, the atoxigenic strain completely or partially colonizes the area, and abundant sporulation provides inoculum levels sufficient to achieve a competitive advantage for the atoxigenic strain.

Some embodiments are directed to a method of controlling aflatoxin contamination in the plant, in a part of the plant, in a product of the plant, etc. The method comprises using the agricultural biocontrol compositions of the invention. In some embodiments, the method is capable of reducing aflatoxin content in the plant, in a part of the plant, or in a product of the plant by at least about 5%, 10%, 15%, 20%, 25%, 30%, 35%, 40%, 45%, 50%, 55%, 60%, 65%, 70%, 75%, 80%, 85%, 90%, 95%, 100%, 2 times, 3 times, 4 times, 5 times, 6 times, 7 times, 8 times, 9 times, 10 times, 20 times, 30 times, 40 times, 50 times, 60 times, 70 times, 80 times, 90 times, 100 times, 200 times, 300 times, 400 times, 500 times, 600 times, 700 times, 800 times, 900 times, 1000 times, or more compared to that in plants not treated with the agricultural biocontrol compositions of the invention.

In some embodiments, the method is capable of reducing aflatoxin content in the plant, in a part of the plant, or in a product of the plant by at least about 5%, 10%, 15%, 20%, 25%, 30%, 35%, 40%, 45%, 50%, 55%, 60%, 65%, 70%, 75%, 80%, 85%, 90%, 95%, 100%, 2 times, 3 times, 4 times, 5 times, 6 times, 7 times, 8 times, 9 times, 10 times, 20 times, 30 times, 40 times, 50 times, 60 times, 70 times, 80 times, 90 times, 100 times, 200 times, 300 times, 400 times, 500 times, 600 times, 700 times, 800 times, 900 times, 1000 times, or more compared to that in plants treated with another agricultural biological composition, such as a biological composition using grain as the nutrient carrier, when the same amount of composition is used.

Yet further embodiments are directed to a method of controlling aflatoxin contamination in a crop related product, or an animal product (where the animal consumes contaminated crops as feed), such as, for example meat, meat product, milk and milk products. The method comprises using the agricultural biocontrol compositions of the invention.

Aflatoxin gets transferred to the milk through contaminated cattle feed (e.g., corn plants), and milk containing 0.5 parts per billion aflatoxin results in milk being dumped. In some embodiments, the method comprises applying the agricultural biocontrol compositions to a field in which cattle feed is grown, or mixing the biocontrol composition with seeds and agricultural plants, at least part of which will be used as cattle feed, before, during, and after the seeds are planted in the field or fed to human or animals. In some embodiments, the method is capable of reducing aflatoxin content in the animal product by at least about 5%, 10%, 15%, 20%, 25%, 30%, 35%, 40%, 45%, 50%, 55%, 60%, 65%, 70%, 75%, 80%, 85%, 90%, 95%, 100%, 2 times, 3 times, 4 times, 5 times, 6 times, 7 times, 8 times, 9 times, 10 times, 20 times, 30 times, 40 times, 50 times, 60 times, 70 times, 80 times, 90 times, 100 times, 200 times, 300 times, 400 times, 500 times, 600 times, 700 times, 800 times, 900 times, 1000 times, or more compared to that in animal products from animals feeding on plants not treated with the agricultural biocontrol compositions of the invention.

In some embodiments, the method is capable of reducing aflatoxin content in the animal product by at least about 5%, 10%, 15%, 20%, 25%, 30%, 35%, 40%, 45%, 50%, 55%, 60%, 65%, 70%, 75%, 80%, 85%, 90%, 95%, 100%, 2 times, 3 times, 4 times, 5 times, 6 times, 7 times, 8 times, 9 times, 10 times, 20 times, 30 times, 40 times, 50 times, 60 times, 70 times, 80 times, 90 times, 100 times, 200 times, 300 times, 400 times, 500 times, 600 times, 700 times, 800 times, 900 times, 1000 times, or more compared to that in animal products derived from an animal feeding on plants treated with another biological composition, such as a biological composition using grain as the nutrient carrier, when the same amount of composition is used.

In some embodiments, the agricultural biocontrol compositions are able to reduce the total content of aflatoxins, and/or the content of one or more particular aflatoxins, such as aflatoxin B1, aflatoxin B2, aflatoxin M1, aflatoxin M2, aflatoxin Q1, aflatoxicol, aflatoxin G1, and aflatoxin G2 in plants growing in the field, in a plant product, in an animal, in an animal product, or in the environment. For aflatoxin biosynthesis, see Ehrlich et al. (2005, Journal of Applied Microbiology 99(3): 518-527), Kusumoto et al. (2000, Current Genetics 37: 104-111), Tominaga et al. (2006, Applied & Environmental Microbiology 72: 484-490), Chang et al. (1995, Molecular & General Genetics 248: 270-277), Lee et al. (2006, Applied Microbiology & Biotechnology 72(2): 339-45), and Wen et al. (2004, Applied and Environmental Microbiology 6: 3192-3198).

In general, starch manufacturing plants procure corn from nearby farmers. Agricultural biocontrol compositions are area-wide management tools that can help protect the area around a manufacturing plant, assuring corn or other target crop is without toxin. This can also reduce transportation cost. Accordingly, the present invention provides a method of controlling aflatoxin contamination in a cultivated area around a starch manufacturing plant. For example, the cultivated area is about 0 mile (0 km), 1 mile (1.6 km), 2 miles (3.2 km), 3 miles (4.8), 4 miles (6.4 km), 5 miles (8.0 km), 10 miles (16.1 km), 15 miles (24.1 km), 20 miles (32.2 km), 25 miles (40.2 km), 30 miles (48.3 km), 35 miles (56.3 km), 40 miles (64.4 km), 45 miles (72.4 km), 50 miles (80.5 km), 55 miles (88.5 km), 60 miles (96.6 km), 70 miles (112 km), 80 miles (129 km), 90 miles (145 km), 100 miles (161 km), or more from the starch manufacturing plant.

It is further disclosed (*not according to the invention*) a method of rapidly producing spores of an *Aspergillus* species, such as an atoxigenic *Aspergillus* strain. The method comprises obtaining corn germ as a nutrient carrier for the sporulation and mixing or combining the corn germ with an *Aspergillus* strain. The *Aspergillus* strain is in the form of spores or active cells. The *Aspergillus* strain is attached to the surface of the corn germ. In some aspects the *Aspergillus* strain is coated on or applied to the surface of the corn germ, for example, through a binding agent or a polymer. The method further comprises sporulating the strain of *Aspergillus* under suitable conditions (e.g., under the proper temperature and moisture).

The method described herein enables rapid production of spores of an *Aspergillus* strain (e.g., an atoxigenic strain) within a short period of time. For example, under suitable conditions, the method produce about 1x10⁶, 2x10⁶, 3x10⁶, 4x10⁶, 5x10⁶, 6x10⁶, 7x10⁶, 8x10⁶, 9x10⁶, 1x10⁷, 2x10⁷, 2x10⁷, 3x10⁷, 4x10⁷, 5x10⁷, 6x10⁷, 7x10⁷, 8x10⁷, 9x10⁷, 1x10⁸, 2x10⁸, 3x10⁸, 4x10⁸, 5x10⁸, 6x10⁸, 7x10⁸, 8x10⁸, 9x10⁸, 1x10⁹, 1x10⁹, 2x10⁹, 3x10⁹, 4x10⁹, 5x10⁹, 6x10⁹, 7x10⁹, 8x10⁹, 9x10⁹, or more spores per gram of the nutrient carrier, within about 24 hours, 36 hours, 48 hours, 60 hours, 72 hours, 84 hours, 96 hours, 108 hours, 120 hours, 132 hours, 144 hours, 156 hours, 168 hours after sporulation begins (e.g., when the agricultural biocontrol composition described herein is placed under the suitable conditions). The yield of spores of a composition can be quantified in the exact same manner by those known in the art. For example, after a standard incubation period at a suitable high humidity (e.g., about 100% relative humidity), spores are washed from the nutrient carrier. The conidial concentration of the spore suspension can be measured with a turbidity meter and calculated with the nephelometric turbidity unit (NTU) versus CFU curve. Usually the average spore yield exceeds about 2-2.5 x 10⁹ spores per gram of the nutrient carrier.

The compositions and methods described herein enable faster production of spores of an *Aspergillus* strain (e.g., an atoxigenic *Aspergillus* strain) compared to compositions and methods using grain as a nutrient carrier for the *Aspergillus* strain, such as those based on rice, wheat, sorghum, barley, etc. For example, the compositions and methods described herein provide at least 5%, 10%, 15%, 20%, 25%, 30%, 35%, 40%, 45%, 50%, 55%, 60%, 65%, 70%, 75%, 80%, 85%, 90%, 95%, 100%, 1.1x, 1.2x, 1.3x, 1.4x, 1.5x, 1.6x, 1.7x, 1.8x, 1.9x, 2x, 2.5x, 3x, 3.5x, 4x, 4.5x, 5x, 5.5x, 6x, 6.5x, 7x, 7.5x, 8x, 8.5x, 9x, 9.5x, 10x, 15x, 20x, 25x, 30x, 35x, 40x, 45x, 50x, 55x, 60x, 65x, 70x, 75x, 80x, 85x, 90x, 95x, 100x, or more spores compared to compositions and methods that use grain as a nutrient carrier under the same conditions, such as those in the commercially available products, including but not limited to, AFLAGUARD^{®}, PREVAIL^{®}, AFLASAFE^{®}, AF-X1^{®}, and FOURSURE^{™}, about 24 hours, 36 hours, 48 hours, 60 hours, 72 hours, 84 hours, 96 hours, 108 hours, 120 hours, 132 hours, 144 hours, 156 hours, 168 hours after sporulation begins.

### Benefits

Existing aflatoxin biocontrol compositions are grain-based biocontrol compositions, which require roasting and cooling before being coated with the beneficial atoxigenic strain. Compared to the existing compositions (e.g., using wheat, sorghum, or barley as a nutrient carrier), compositions and methods described herein achieve the following benefits:
1. Compositions and methods of the invention provide a higher spore yield per gram of nutrient carrier. Most biocontrol compositions worldwide use sorghum as the nutrient carrier (e.g., AFLASAFE^{®} developed by IITA and USDA). Wheat and barley are also used (e.g., AFLAGUARD^{®} developed by USDA and distributed by Syngenta, and PREVAIL^{®} distributed by Arizona Cotton Research and Protection Council). Compared to these compositions and related manufacturing processes, the agricultural biocontrol compositions of the invention produce many more spores per gram of nutrient carrier. For example, the agricultural biocontrol composition can produce many more spores within 48 hours per gram nutrient carrier comprising corn germ compared to a composition contains sorghum as the nutrient carrier. Therefore, the agricultural biocontrol compositions of the invention ensure atoxigenic genotypes are actively growing in the field prior to the time when aflatoxin-producing strains rapidly increase. Due to the rapid sporulation of the beneficial atoxigenic *Aspergillus* strain after application, a large amount of atoxigenic spores are produced and protect the crop by displacing and competing with aflatoxin producers. Furthermore, the fast sporulation reduces the damage to the nutrient carrier or removal of the nutrient carrier through predation by birds, mammals (e.g. rodents) or insects before optimum quantity of spores are produced.
2. Since the corn germ contained in the agricultural biocontrol compositions of the invention can be produced through the corn wet-milling process, which is a well-established starch purification process that includes steeping and drying at high temperature, compositions of the present disclosure have a reliable microbiology profile (e.g., the microbes on the germ). For example, the compositions do not contain unwanted fungi and bacteria (e.g., enterobacteria), or only contains very low levels of unwanted fungi or bacteria, which would not raise concern (e.g., undetectable, or meeting the requirements set by EPA).
3. Compositions and methods of the invention require only size sorting and coating equipment when the corn germ is produced for making the agricultural biocontrol compositions described herein. Since no grain storage and roasting equipment are required, the compositions and methods lead to less capital investment. It is less expensive also because less energy and labor are required for using germ as the nutrient carrier.
4. Compositions and methods of the invention require lower starting cost, and less labor thus leading to very low cost of production. For example, very few people are required for manufacture and quality control. Lower cost encourages farmers to apply biocontrol product preemptively, which changes fungal population resulting in area-wide reductions in aflatoxins. This can be especially important for processing or manufacturing plants that must draw crop from throughout an area. Further, since packaging is the most cost intensive portion, bulk packaging is acceptable in some embodiments.
5. Compositions and methods of the invention enable the processes for manufacturing the agricultural biocontrol compositions described herein to be more efficient, simpler, and require less energy than existing biocontrol agent manufacturing processes. For example, no pearling, roasting, or steaming step is required in the manufacturing process described herein, which leads to lower utility cost.
6. Compositions of the invention can be produced and used in close proximity to farming communities that require aflatoxin management, including those providing corn plants to starch manufacturing facilities. For example, there are starch plants in many locations. Compositions can be directly produced within or near the starch plants as a by-product of a corn wet-milling process and applied directly to the area where corn is produced, which in turn reduces shipping cost.
7. Making and using compositions of the invention does not generate additional waste. For example, removed materials are used to produce oil and feed, in a similar manner to which unsorted germ is used. In addition, no hulls and no material resulting from grain cleaning are produced. Since corn germ is already being produced by a starch manufacturing plant as a co-product, it is readily available for use without further or very little processing.
8. Making and using compositions of the invention do not use a directly consumable food. For example, corn germ in general is not a directly consumable food, and must be extracted to make oil plus feed. This is unlike wheat, barley, and sorghum (all three are used as food in some regions), as even though very little grain of wheat, barley, or sorghum is used, users, NGOs, and government entities may complain about food quality grains being used to produce biocontrol products.
9. In certain embodiments, corn germ is produced as a co-product of a starch manufacturing process, which can only be supplied by a starch plant. Therefore, the process of the invention results in biocontrol compositions described herein being available in regions where starch manufacturing plants are present.

In some embodiments, the current invention includes germ purification and particle sizing that allow consistent product size over time and deliberate management of particle size to enhance flowability and to optimize the number of product particles per mass and thus flexibility to manage the number of particles per weight delivered to treated fields in order to optimize coverage of target crops.

In addition, compositions and methods of the invention enable faster and greater sporulation of beneficial atoxigenic *Aspergillus* strains for aflatoxin biocontrol, while effectively reducing labor and cost compared to existing commercial biocontrol compositions that contain grains as the nutrient carriers. For example, biocontrol compositions that contain cereal grains (e.g., wheat, sorghum, barley, etc.) require several steps. Particularly, grains must be purchased, transported to the facility, stored, pasteurized, and cooled before coating with the beneficial atoxigenic spore suspension. These steps require more labor, extensive capital investment in equipment, space and lead to higher operating cost due to energy use. However, these steps are not required to produce the agricultural biocontrol compositions described herein.

In summary, using germ as the nutrient carrier simplifies the manufacturing process, which now requires only sizing and coating equipment, making it more efficient and less labor intensive. While biocontrol technology assures safe food, germ based formulations add to sustainability of the technology by using a coproduct, reducing energy cost, eliminating waste, and making the product more effective. Overall, using germ reduces cost of biocontrol product by eliminating or minimizing equipment and energy cost, while contributing towards a more effective product via faster and more sporulation.

### Definitions

References to "one embodiment", "an embodiment", "one example", and "an example" indicate that the embodiment(s) or example(s) so described may include a particular feature, structure, characteristic, property, element, or limitation, but that not every embodiment or example necessarily includes that particular feature, structure, characteristic, property, element or limitation. Furthermore, repeated use of the phrase "in one embodiment" does not necessarily refer to the same embodiment, though it may.

As used herein, the term "about" refers to plus or minus 10% or 5% of the referenced number.

The phrase "consisting essentially of" means that the composition or method may include additional ingredients and/or steps, but only if the additional ingredients and/or steps do not materially alter the basic and novel characteristics of the claimed composition or method.

As used herein, the singular form "a", "an" and "the" include plural references unless the context clearly dictates otherwise. For example, the term "a compound" or "at least one compound" may include a plurality of compounds, including mixtures thereof.

The word "exemplary" is used herein to mean "serving as an example, instance or illustration". Any embodiment described as "exemplary" is not necessarily to be construed as preferred or advantageous over other embodiments and/or to exclude the incorporation of features from other embodiments.

The word "optionally" is used herein to mean "is provided in some embodiments and not provided in other embodiments". Any particular embodiment of the invention may include a plurality of "optional" features unless such features conflict.

Certain embodiments of the present disclosure are further described in the following Examples. It should be understood that these Examples are given by way of illustration only.

### EXAMPLES

### Example 1

### Production of an Aflatoxin Biocontrol Composition

Corn germ was isolated during wet-milling process, which gave germ a reliable microbiology resulting from well-established starch purification process that included steeping and drying. Germ did not have unwanted fungi and did not need to be pasteurized reducing equipment and energy cost. This germ was cleaned to remove impurities (pericarp etc.) which don't support fungal growth, and sieved (7 Mesh) for right size removing broken pieces. This cleaned, sieved germ was coated with a beneficial atoxigenic *Aspergillus* strain and a polymer (Treating Solutions Green Polymer 3118, Milliken & Co.) to create an aflatoxin biocontrol composition.

### Example 2

### Efficacy of the Aflatoxin Biocontrol Composition in Promoting Sporulation

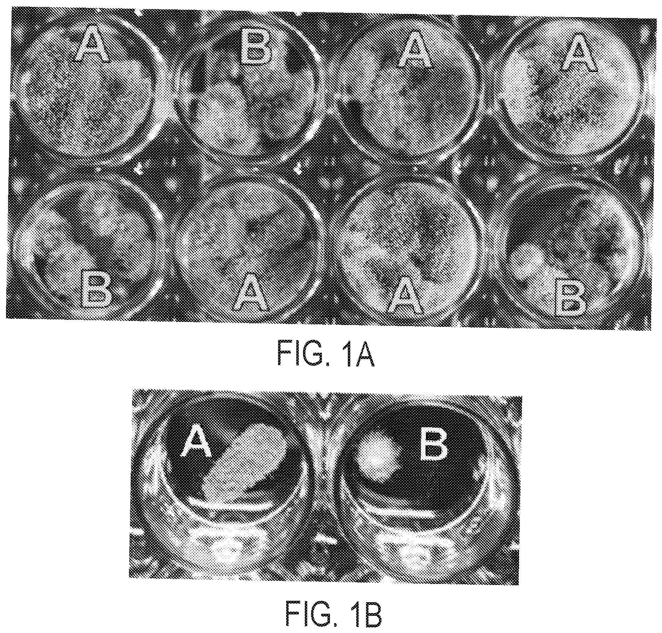

Corn germ as the nutrient carrier was compared with sorghum as the nutrient carrier in order to assess the ability of promoting sporulation of an atoxigenic *Aspergillus* strain. Essentially, the same amount of an atoxigenic *Aspergillus* strain was inoculated to corn germ or sorghum. After 48 hours after being incubated under conditions suitable for sporulation, sporulation status was checked and recorded, up to 7 days. The result as shown in Table 1 and Figure 1 clearly indicates that much more spores (represented as Nephelometric Turbidity Units (NTU)) were produced on the surface of corn germ compared to sorghum.

| **Table 1: Comparison of Spore Production on Germ and Sorghum by Atoxigenic *A*. *flavus,* average of three replications** | | | | |
|---|---|---|---|---|
| **Experiment** | **Time (h)** | **Spore Yield (Spores/g)** | | **Fold Increase (Corn Germ vs. Sorghum)** |
| | | **Sorghum** | **Corn Germ** | |
| 1 | 48 | 5.8 x 10⁶ | 9.3 x 10⁷ | 16x |
| | 72 | 5.7 x 10⁷ | 5.5 x 10⁸ | 10x |
| | 96 | 4.0 x 10⁸ | 1.1 x 10⁹ | 3x |
| 2 | 48 | 2.6 x 10⁶ | 1.1 x 10⁸ | 43x |
| | 96 | 1.6 x 10⁸ | 4.5 x 10⁸ | 3x |
| | 168 | 4.5 x 10⁸ | 7.1 x 10⁸ | 2x |

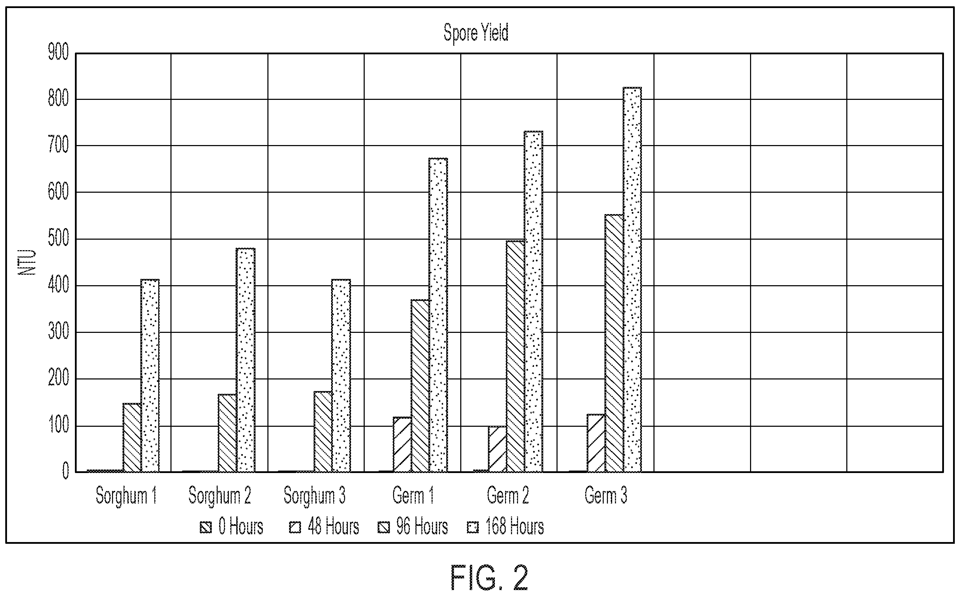

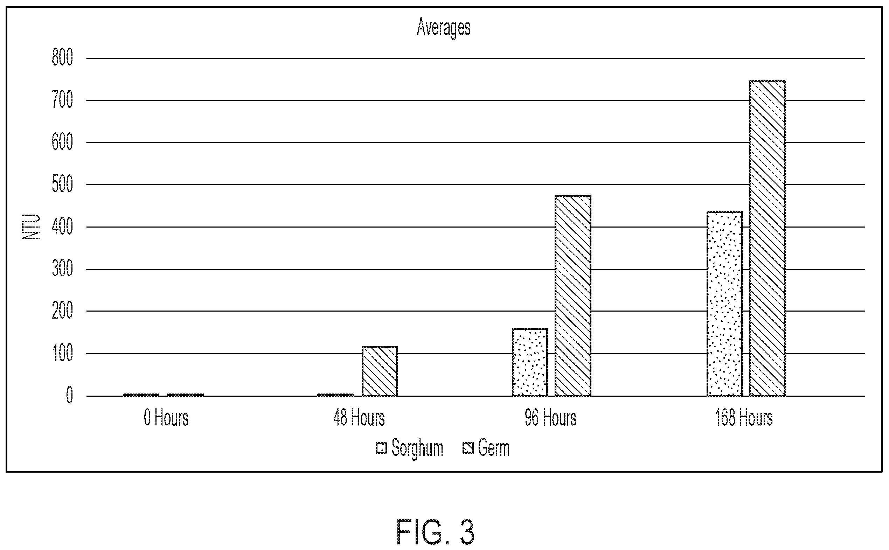

Next, sorghum and corn germ were used as the nutrient carrier to test their ability of promoting sporulation of an atoxigenic *Aspergillus* strain. Each was inoculated with the same amount of the atoxigenic *Aspergillus* strain with three replicas. The amount of induced spores was measured as Nephelometric Turbidity Units (NTU) within 0-168 hours after the inoculation. The results as shown in Table 2A and Figure 2 and Figure 3 confirm again that the corn germ produced much more spores compared to sorghum from early on (e.g., within 48 hours after inoculation). Table 2B demonstrates Corn Germ outperforming Sorghum.

| **Table 2A: Comparison in Spore Yield of Sorghum vs Germ (0-168 hours, NTU)** | | | | |
|---|---|---|---|---|
| | **0 Hours** | **48 Hours** | **96 Hours** | **168 Hours** |
| **Sorghum 1** | 3.29 | 2.31 | 145 | 412 |
| **Sorghum 2** | 3.28 | 2.05 | 165 | 481 |
| **Sorghum 3** | 1.44 | 3.01 | 169 | 409 |
| **Germ 1** | 2.58 | 118 | 368 | 669 |
| **Germ 2** | 2.32 | 99.3 | 492 | 730 |
| **Germ 3** | 2.03 | 124 | 552 | 828 |

| **Table 2B: Performance of Atoxigenic *Aspergillus* Strains as Active Ingredients When Combined with a Corn Germ or Sorghum Nutrient Carrier After 7 Days (168 hours)** | | | | | | |
|---|---|---|---|---|---|---|
| | **Spore Yield per Particle** | | **Fold Increase** | **Spore Yield per Gram** | | **Fold Increase** |
| **Fungus strains (identified by deposit number)¹** | Sorghum | Corn Germ | Corn Germ vs Sorghum | Sorghum | Corn Germ | Corn Germ vs Sorghum |
| **1. NRRL 18543²** | 5.94E+06 | 7.03E+06 | 1.18 | 2.07E+08 | 3.01E+08 | 1.46 |
| **2. NRRL 21882³** | 3.23E+06 | 6.14E+06 | 1.90 | 1.12E+08 | 2.63E+08 | 2.34 |
| **3. FGSC A2220** | 4.89E+06 | 8.96E+06 | 1.83 | 1.70E+08 | 3.84E+08 | 2.26 |
| **4. FGSC A2223** | 4.97E+06 | 7.42E+06 | 1.49 | 1.73E+08 | 3.18E+08 | 1.84 |
| **5. FGSC A2226** | 3.84E+06 | 5.15E+06 | 1.34 | 1.34E+08 | 2.21E+08 | 1.65 |
| **6. FGSC A2229** | 4.60E+06 | 7.90E+06 | 1.72 | 1.60E+08 | 3.38E+08 | 2.11 |
| **7. Mixture of strains 3 to 6⁴** | 2.48E+06 | 6.42E+06 | 2.59 | 8.61E+07 | 2.75E+08 | 3.19 |

| | | | | | | |
|---|---|---|---|---|---|---|
| 1. The NRRL fungi are deposited in the ARS Culture Collection, FGSC fungi are deposited in the Fungal Genetic Stock Center. 2. Atoxigenic genotype contained in AF36 Prevail (Arizona Cotton Research and Protection Council trademark). 3. Atoxigenic genotype of Afla-guard^{®}(Syngenta)(AFLA-GUARD is a registered trademark of Circle-One Global, Inc.). 4. Atoxigenic genotypes contained in equal quantities in FourSure^{®} (Texas Corn Producers Board) (FOURSURE is a registered trademark of Texas Corn Producers Board). | | | | | | |

### Example 3

### Efficacy of an Aflatoxin Biocontrol Composition in Reducing the Content of Aflatoxin in Maize Grain

### Efficacy of Atoxigenic Aspergillus flavus strain of AflaPak in Laboratory Tests

Efficacy of the *Aspergillus flavus* strain in AflaPak was assessed in laboratory studies on mature maize kernels. The type of assay used in this study also reflects increases in aflatoxin that occur in the field when it rains on the mature crop or when both high humidity and high temperature exist after maturation. This type of assay also reflects efficacy that is relevant after the crop has been harvested and maize with moisture content above 15% is transported, stored, or even mixed into feed and fed (i.e. in the yard at a dairy).

Laboratory tests were performed utilizing the same methods that were used for the initial screens of the atoxigenic genotypes of *A. flavus* currently being used in farmer fields in Kenya where the most severe effects of aflatoxins on humans are known. Details of the methods are in Probst et al. 2011 ("Identification of atoxigenic Aspergillus flavus isolates to reduce aflatoxin contamination of maize in Kenya." Plant Disease, Vol. 95, No. 2, pp 212-218). Results and statistical analyses of laboratory tests on maize are shown in Table 3. Table 3 shows that the *A. flavus* strain in AflaPak reduces aflatoxin contamination caused by all four high aflatoxin-producing fungi strains (PKM31-H, PKM30-G, PKM11-L, and PKM62-D) isolated from maize in Pakistan.

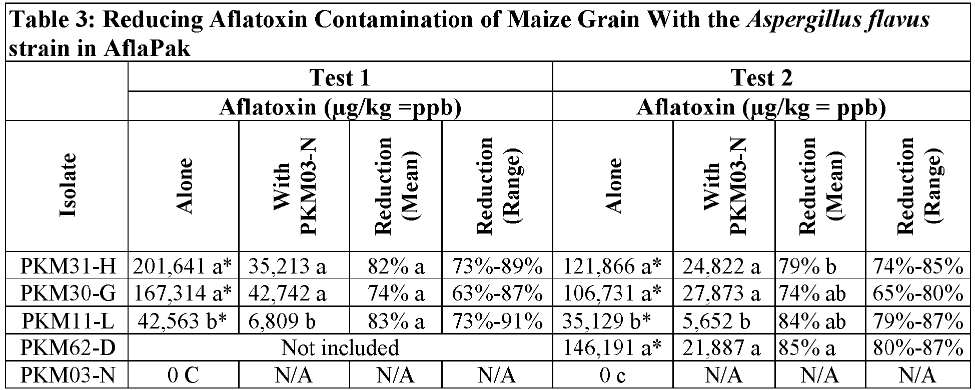

Both tests were performed on whole maize kernels following procedures in Probst et al. 2011 and Probst and Cotty, 2012. Values are means of four replicates. All fungi were isolated from maize produced in Pakistan. PKM3 1-H, PKM30-G, PKM11-L, and PKM03-N belong to the L-strain morphotype of *A. flavus.* PKM62-D belongs to the S-strain morphotype of *A. flavus.* The strain in AflaPak is missing genes required for aflatoxin biosynthesis and, as a result, it is an atoxigenic genotype. Atoxigenic means it does not have the capacity to produced aflatoxins and is useful for aflatoxin mitigation.

Mention of any reference, article, publication, patent, patent publication, and patent application cited herein is not, and should not, be taken as an acknowledgment or any form of suggestion that they constitute valid prior art or form part of the common general knowledge in any country in the world.

Unless defined otherwise, all technical and scientific terms herein have the same meaning as commonly understood by one of ordinary skill in the art to which this invention belongs.

The publications discussed herein are provided solely for their disclosure prior to the filing date of the present application. Nothing herein is to be construed as an admission that the present invention is not entitled to antedate such publication by virtue of prior invention.

## Claims

1. An agricultural biocontrol composition comprising an atoxigenic strain of *Aspergillus,* and a nutrient carrier, wherein the nutrient carrier comprises corn germ, and wherein the atoxigenic strain of *Aspergillus* is coated on the surface of the corn germ.

2. The agricultural biocontrol composition of claim 1, wherein the composition further comprises one or more elements selected from the group consisting of a carrier agent, a spreading agent (spreader), a binding agent, an osmoprotectant, a colorant, and a preservative.

3. The agricultural biocontrol composition of claim 1 or 2, wherein the composition further comprises a seed binder optionally comprising a polymer.

4. The agricultural biocontrol composition according to any one of claims 1-3, wherein the corn germ is produced as a by-product of a corn wet-milling process.

5. The agricultural biocontrol composition according to any one of claims 1-4, wherein the corn germ is size sorted with sieving means, wherein a set of sieves of specified sizes are used to remove smaller and larger pieces and provide a desired size of germ particles, and wherein U.S. Sieve No. 7 or No. 8 is used to retain the desirable size and allow smaller pieces through, and U.S. Sieve No. 6 is used to retain larger undesirable pieces and trash.

6. A method for producing an agricultural biocontrol composition according to claim 1, comprising:
(1) obtaining corn germ; and
(2) combining the corn germ with an atoxigenic strain of *Aspergillus* to produce the agricultural biocontrol composition.

7. The method of claim 6, wherein the corn germ is produced as a by-product of a corn wet-milling process.

8. The method of claim 6 or 7, wherein the corn germ is sieved to remove pieces of undesirable size.

9. The method according to any one of claims 6-8, wherein the method comprises:
(1) producing corn germ as a by-product of a corn wet-milling process;
(2) sieving the corn germ produced in step (1) to remove broken pieces;
(3) combining the sieved corn germ with an atoxigenic strain of *Aspergillus* to produce the agricultural biocontrol composition.

10. The composition according to any one of claim 1-5 or the method according to any one of claims 6-9, wherein the atoxigenic strain of *Aspergillus* is an *Aspergillus oryzae* strain, an *Aspergillus flavus* strain, an *Aspergillus sojae* strain, or a mixture thereof.

11. The method according to any one of claims 6-10, wherein the agricultural biocontrol composition is essentially free of fungi other than the atoxigenic strain of *Aspergillus,* and essentially free of disease-causing enterobacteria.

12. A method for controlling aflatoxin contamination comprising applying to an agricultural plant, an agricultural product derived from said plant, or a cultivated area an aflatoxin-reducing effective amount of the agricultural biocontrol composition according to any one of claims 1-5 to the plant, plant product or cultivate area.

13. The method of claim 12, wherein the agricultural biocontrol composition is in a water-dispersible granular formulation.

## Patentansprüche

1. Zusammensetzung für eine landwirtschaftliche biologische Schädlingsbekämpfung, umfassend einen atoxigenen Stamm von *Aspergillus* und einen Nährstoffträger, wobei der Nährstoffträger einen Maiskeim umfasst und wobei der atoxigene Stamm von *Aspergillus* auf die Oberfläche des Maiskeims aufgetragen ist.

2. Zusammensetzung für eine landwirtschaftliche biologische Schädlingsbekämpfung nach Anspruch 1, wobei die Zusammensetzung ferner ein oder mehrere Elemente umfasst, die aus der Gruppe ausgewählt sind, bestehend aus einem Trägermittel, einem Spreitmittel (Spreitstoff), einem Bindemittel, einem Osmoschutzmittel, einem Farbstoff und einem Konservierungsmittel.

3. Zusammensetzung für eine landwirtschaftliche biologische Schädlingsbekämpfung nach Anspruch 1 oder 2, wobei die Zusammensetzung ferner einen Saatgutbinder umfasst, optional umfassend ein Polymer.

4. Zusammensetzung für eine landwirtschaftliche biologische Schädlingsbekämpfung nach einem der Ansprüche 1 bis 3, wobei der Maiskeim als ein Nebenprodukt eines Maisnassmahlprozesses produziert wird.

5. Zusammensetzung für eine landwirtschaftliche biologische Schädlingsbekämpfung nach einem der Ansprüche 1 bis 4, wobei der Maiskeim mit Siebmitteln nach Größe sortiert wird, wobei ein Satz von Sieben spezifischer Größe verwendet wird, um kleinere und größere Stücke zu entfernen und eine gewünschte Größe von Keimpartikeln bereitzustellen, und wobei ein US-Sieb Nr. 7 oder Nr. 8 verwendet wird, um die gewünschte Größe zurückzuhalten und kleinere Stücke durchzulassen, und das US-Sieb Nr. 6 verwendet wird, um größere, unerwünschte Stücke und Abfall zurückzuhalten.

6. Verfahren zum Produzieren einer Zusammensetzung für eine landwirtschaftliche biologische Schädlingsbekämpfung nach Anspruch 1, umfassend:
(1) Erhalten des Maiskeims; und
(2) Kombinieren des Maiskeims mit einem atoxigenen Stamm von *Aspergillus,* um die Zusammensetzung für eine landwirtschaftliche biologische Schädlingsbekämpfung zu produzieren.

7. Verfahren nach Anspruch 6, wobei der Maiskeim als ein Nebenprodukt eines Maisnassmahlprozesses produziert wird.

8. Verfahren nach Anspruch 6 oder 7, wobei der Maiskeim gesiebt wird, um Stücke unerwünschter Größe zu entfernen.

9. Verfahren nach einem der Ansprüche 6 bis 8, wobei das Verfahren umfasst:
(1) Produzieren des Maiskeims als ein Nebenprodukt eines Maisnassmahlprozesses;
(2) Sieben des Maiskeims, der in Schritt (1) produziert wird, um Bruchstücke zu entfernen;
(3) Kombinieren des gesiebten Maiskeims mit einem atoxigenen Stamm von *Aspergillus,* um die Zusammensetzung für eine landwirtschaftliche biologische Schädlingsbekämpfung zu produzieren.

10. Zusammensetzung nach einem der Ansprüche 1 bis 5 oder Verfahren nach einem der Ansprüche 6 bis 9, wobei der atoxigene Stamm von *Aspergillus* ein Stamm *Aspergillus oryzae,* ein Stamm *Aspergillus flavus,* ein Stamm *Aspergillus sojae* oder eine Mischung davon ist.

11. Verfahren nach einem der Ansprüche 6 bis 10, wobei die Zusammensetzung für eine landwirtschaftliche biologische Schädlingsbekämpfung im Wesentlichen frei von Pilzen, mit Ausnahme des atoxigenen Stamms von *Aspergillus,* und im Wesentlichen frei von krankheitserregenden Enterobakterien ist.

12. Verfahren zum Bekämpfen einer Aflatoxinkontamination, umfassend ein Aufbringen, auf eine landwirtschaftliche Pflanze, ein aus der Pflanze gewonnenes landwirtschaftliches Produkt oder eine Anbaufläche, einer aflatoxinreduzierenden wirksamen Menge der Zusammensetzung für eine landwirtschaftliche biologische Schädlingsbekämpfung nach einem der Ansprüche 1 bis 5 auf die Pflanze, das Pflanzenprodukt oder die Anbaufläche.

13. Verfahren nach Anspruch 12, wobei die Zusammensetzung für eine landwirtschaftliche biologische Schädlingsbekämpfung in einer wasserdispergierbaren Granulatformulierung vorliegt.

## Revendications

1. Composition de biocontrôle agricole comprenant une souche atoxinogène *d'Aspergillus,* et un support nutritif, dans laquelle le support nutritif comprend du germe de maïs, et dans laquelle la souche atoxinogène *d'Aspergillus* est revêtue sur la surface du germe de maïs.

2. Composition de biocontrôle agricole selon la revendication 1, dans laquelle la composition comprend en outre un ou plusieurs éléments choisis dans le groupe constitué par un agent de support, un agent d'épandage (épandeur), un agent de liaison, un osmoprotecteur, un colorant et un conservateur.

3. Composition de biocontrôle agricole selon la revendication 1 ou 2, dans laquelle la composition comprend en outre un liant de semence comprenant facultativement un polymère.

4. Composition de biocontrôle agricole selon l'une quelconque des revendications 1 à 3, dans laquelle le germe de maïs est produit en tant que sous-produit d'un processus de mouture humide de maïs.

5. Composition de biocontrôle agricole selon l'une quelconque des revendications 1 à 4, dans laquelle le germe de maïs est trié par taille avec un moyen de tamisage, dans laquelle un ensemble de tamis de tailles spécifiées est utilisé pour éliminer les morceaux plus petits et plus grands et fournir une taille désirée de particules de germe, et dans laquelle un tamis américain No. 7 ou No. 8 est utilisé pour retenir la taille désirée et laisser passer les morceaux plus petits, et un tamis américain No. 6 est utilisé pour retenir les morceaux indésirables plus grands et les déchets.

6. Procédé permettant de produire une composition de biocontrôle agricole selon la revendication 1, comprenant :
(1) l'obtention de germe de maïs ; et
(2) la combinaison du germe de maïs avec une souche atoxinogène *d'Aspergillus* pour produire la composition de biocontrôle agricole.

7. Procédé selon la revendication 6, dans lequel le germe de maïs est produit en tant que sous-produit d'un processus de mouture humide de maïs.

8. Procédé selon la revendication 6 ou 7, dans lequel le germe de maïs est tamisé pour éliminer les morceaux de taille indésirable.

9. Procédé selon l'une quelconque des revendications 6 à 8, dans lequel le procédé comprend :
(1) la production de germe de maïs en tant que sous-produit d'un processus de mouture humide de maïs ;
(2) le tamisage du germe de maïs produit à l'étape (1) pour éliminer les morceaux cassés ;
(3) la combinaison du germe de maïs tamisé avec une souche atoxinogène *d'Aspergillus* pour produire la composition de biocontrôle agricole.

10. Composition selon l'une quelconque des revendications 1 à 5 ou procédé selon l'une quelconque des revendications 6 à 9, dans lequel la souche atoxinogène *d'Aspergillus* est une souche d'*Aspergillus oryzae,* une souche d' *Aspergillus flavus,* une souche d'*Aspergillus sojae,* ou un mélange de celles-ci.

11. Procédé selon l'une quelconque des revendications 6 à 10, dans lequel la composition de biocontrôle agricole est sensiblement dépourvue de champignons autres que la souche atoxinogène *d'Aspergillus,* et sensiblement dépourvue d'entérobactéries pathogènes.

12. Procédé permettant de contrôler une contamination par aflatoxine comprenant l'application à une plante agricole, à un produit agricole dérivé de ladite plante, ou à une zone cultivée, d'une quantité efficace pour réduire l'aflatoxine de la composition de biocontrôle agricole selon l'une quelconque des revendications 1 à 5 à la plante, au produit de plante ou à la zone cultivée.

13. Procédé selon la revendication 12, dans lequel la composition de biocontrôle agricole est dans une formulation granulaire hydrodispersible.
